# FASCICULE DE BREVET EUROPEEN

(11) **EP 3 563 139 B1**
(45) Date de publication et mention de la délivrance du brevet: **24.03.2021**
(21) Numéro de dépôt: 17822410.1
(22) Date de dépôt: 13.12.2017
(51) Int. Cl.: G01N 1/40, G01N 33/68, H01J 49/04

(54) **PROCÉDÉ DE LIBÉRATION ET DE CAPTURE SPATIALISÉES D'ESPÈCES BIOLOGIQUES À PARTIR D'UN TISSU DÉPOSÉ SUR UN SUPPORT FONCTIONNALISÉ**
VERFAHREN ZUR BEABSTANDETEN BEFREIUNG UND ERFASSUNG VON BIOLOGISCHEN SPEZIES UNTER VERWENDUNG EINES GEWEBES AUF EINEM FUNKTIONALISIERTEN TRÄGER
METHOD OF SPATIALIZED FREEING AND CAPTURING OF BIOLOGICAL SPECIES USING A TISSUE PLACED ON A FUNCTIONALIZED SUPPORT

(30) Priorité: 29.12.2016 FR 1663528
(43) Date de publication de la demande: 06.11.2019
(73) Titulaire: Commissariat à l'Energie Atomique et aux Energies Alternatives, 75015 Paris (FR)
(72) Inventeur: MOMBRUN, Adrien, 38000 Grenoble (FR); BOUAMRANI, Mohamed-Ali, 38000 Grenoble (FR); LE CLEC'H, Céline, 38470 Têche (FR)
(74) Mandataire: INNOV-GROUP
(86) Numéro de dépôt international: PCT/FR2017/053545
(87) Numéro de publication internationale: WO 2018/122487

(56) Documents cités:
- WO-A1-2013/098703
- GB-A- 2 437 623
- US-A1- 2015 093 780

## Description

### DOMAINE TECHNIQUE

Le domaine technique de l'invention est la libération et la capture spatialisée et sélective d'espèces biologiques, par exemple des protéines ou des peptides, à partir d'un tissu, par exemple une coupe de tissu histologique, déposé sur un support fonctionnalisé, par exemple du silicium nanoporeux.

### ART ANTERIEUR

L'analyse histologique ou immunohistochimique en anatomopathologie consiste à analyser des échantillons de tissu biologique de faible épaisseur, pour établir un diagnostic. L'échantillon de tissu se présente généralement sous la forme d'une fine coupe, obtenue selon des procédés de préparation connus, à partir d'un prélèvement effectué par exérèse, biopsie ou frottis. La préparation consiste à former une coupe de fine épaisseur déposée sur un support, généralement un support transparent. Avant la découpe, le tissu peut être congelé tel quel ou fixé chimiquement, déshydraté et inclus dans une matrice de paraffine. L'analyse histologique de la coupe de tissu est réalisée en routine par des colorations histologiques, traditionnellement l'hématoxyline et l'éosine, de façon à déterminer la topographie du tissu. La coupe peut aussi être analysée par immunohistochimie, faisant appel à des anticorps ciblés sur des protéines connues comme étant des biomarqueurs d'une pathologie donnée de manière à affiner le diagnostic clinique. Cependant, ces techniques sont sujettes à l'appréciation du praticien, la spécificité/sensibilité des anticorps, et la pertinence des biomarqueurs choisis. Elles peuvent s'avérer insuffisantes et non pertinentes pour un diagnostic objectif et spécifique d'une pathologie donnée, et sont inadaptées à la recherche de nouveau biomarqueurs pour le diagnostic clinique.

Le document US2015/0093780 décrit un procédé de capture par pulvérisation d'une matrice sur une lame de tissu, la matrice ayant la capacité de se lier à des analytes du tissu.

Récemment, l'imagerie moléculaire par spectrométrie de masse, et notamment l'imagerie MALDI-TOF (Matrix Assisted Laser Desorption Ionisation - Time of Flight), s'est révélée comme une nouvelle alternative pour l'analyse moléculaire non supervisée de coupes histologiques de tissus biologiques. Elle permet de déterminer la répartition spatiale, de manière semi-quantitative, de molécules organiques et/ou inorganiques (par exemple : métabolites, peptides, protéines, lipides, molécules pharmaceutiques). Cependant la sensibilité de la technique est limitée par la présence de molécules majoritaires et abondantes, non pertinentes pour le diagnostic, la recherche de biomarqueurs, ou la détection d'une molécule spécifique. Notamment, la gamme de concentration et le nombre de molécules présentes dans un échantillon biologique provoquent une perte de sensibilité de l'analyse pour la détection de molécules potentiellement intéressantes, c'est-à-dire les biomarqueurs. Il est donc nécessaire de fractionner l'échantillon, de manière à le décomplexifier, et ainsi améliorer la sensibilité de la détection, ainsi que le nombre de molécules détectées sur un même échantillon.

La demande WO2013/098703 décrit un support de capture en silicium nanoporeux, destiné à être apposé sur un tissu. Sous l'effet de la porosité, le support permet de prélever sélectivement des protéines de faible masse, ces dernières se fixant dans les pores. Après rinçage, les protéines prélevées restent confinées au niveau des pores, tandis que les protéines de grande taille sont éliminées. Le support étant conducteur, il est compatible avec l'imagerie moléculaire par spectrométrie de masse MALDI, de façon à obtenir une distribution spatiale des molécules d'intérêt. Un tel support constitue donc un outil particulièrement prometteur pour l'enrichissement spatialisé de molécules de faible poids moléculaire d'un tissu biologique. De plus les performances des spectromètres de masse MALDI (sensibilité, résolution spectrale) sont optimales en dessous de 20000 Da, c'est-à-dire pour les bas poids moléculaires. Le procédé ainsi décrit permet une meilleure sensibilité de détection ainsi qu'un plus grand nombre de molécules détectées, chacune ayant le potentiel d'être un marqueur pertinent d'une pathologie donnée.

Les inventeurs de la présente invention ont utilisé le support de capture similaire à celui décrit dans cette demande WO2013/098703. Ils ont mis au point un procédé d'analyse particulièrement adapté à des coupes de tissus histologiques. Le procédé permet d'effectuer une analyse performante de coupes de tissus, et constitue une alternative séduisante aux méthodes usuellement mises en oeuvre dans les analyses histologiques.

### EXPOSE DE L'INVENTION

Un objet de l'invention est un procédé de capture d'espèces biologiques à partir d'un tissu biologique, comportant les étapes suivantes :
a) application d'un tissu biologique sur un support de capture, le support de capture étant apte à capturer sélectivement au moins une espèce biologique, dite espèce biologique d'intérêt ;
b) dépôt d'un réactif de lyse sur le tissu biologique ;
c) mise en contact du réactif de lyse, ainsi déposé, avec un solvant, de façon à former des gouttelettes, espacées les unes des autres, à la surface du tissu, les gouttelettes comportant le réactif de lyse solubilisé par le solvant;
d) formation de sites de lyse, chaque site de lyse correspondant à une portion du tissu s'étendant entre une gouttelette et le support de capture et, dans chaque site de lyse, lyse du tissu par le réactif de lyse solubilisé par le solvant, la lyse entraînant une libération puis une capture des espèces biologiques d'intérêt du tissu par le support de capture ;
e) rinçage du support de capture, pour éliminer les espèces biologiques non capturées.

Le procédé peut également comporter une étape f) d'analyse spatialement résolue du support nanoporeux, de manière à obtenir une information spatiale relative aux espèces biologiques capturées lors de l'étape d). L'analyse mise en œuvre est généralement une spectrométrie de masse spatialement résolue, par exemple une spectrométrie de masse par désorption laser.

Selon un mode de réalisation, lors de l'étape b), le réactif de lyse peut être pulvérisé sur le tissu. Selon un mode de réalisation, lors de l'étape b) le réactif de lyse se condense sur le tissu, de façon à former des dépôts solides ,ou cristaux, sur ce dernier. Selon ce mode de réalisation, le procédé peut comporter une sublimation du réactif de lyse, de telle sorte que le réactif de lyse, à l'état gazeux, se condense sur le tissu.

Selon un mode de réalisation, lors de l'étape c), les gouttelettes sont formées par condensation du solvant sur le tissu. Selon un autre mode de réalisation, lors de l'étape c), les gouttelettes sont formées par pulvérisation du solvant sur le tissu.

Le support de capture peut être un support nanoporeux, comportant de préférence des pores dont le diamètre ou la plus grande taille est inférieur à 1 µm. Le support de capture peut être en silicium. Il peut également s'agir d'un support de capture fonctionnalisé par dépôt d'une couche apte à retenir des espèces biologiques d'intérêt.

Le tissu est de préférence disposé selon une coupe. L'épaisseur de tissu peut être inférieure à 50 µm ou à 20 µm.

D'autres avantages et caractéristiques ressortiront plus clairement de la description qui va suivre de modes particuliers de réalisation de l'invention, donnés à titre d'exemples non limitatifs, et représentés sur les figures listées ci-dessous.

### FIGURES

Les figures 1A, 1B, 1C et 1D représentent différentes phases d'un procédé selon l'invention.
La figure 2A schématise les principales étapes d'un procédé selon l'invention. La figure 2B représente une vue au microscope d'une coupe de tissu brut avant mise en œuvre du procédé.
La figure 2C représente une vue au microscope d'une coupe de tissu après obtention d'une lyse dite spatialisée, selon un mode de réalisation dans lequel le réactif de lyse est sublimé et condensé sur le tissu, puis dans lequel une lyse est activée par incubation dans une chambre saturée en vapeurs de solvant. La figure 2D représente une vue au microscope d'une coupe de tissu après obtention d'une lyse spatialisée selon une variante selon laquelle le réactif de lyse est pulvérisé sur le tissu, sous forme liquide, puis la lyse est activée par pulvérisation d'un solvant.
La figure 3 montre un spectre acquis en mettant en œuvre le procédé décrit en lien avec la figure 2A respectivement sur un support lisse d'oxyde d'indium-étain utilisé en tant que support de référence, et sur un support en silicium nanoporeux.
Les figures 4A et 4B représentent respectivement une distribution spatiale de l'intensité d'un pic correspondant à une masse de 4192 Da, résultant de spectres de masse obtenus sur une lame de tissu déposée sur un support de référence et sur un support nanoporeux.
La figure 5A représente une distribution spatiale de l'intensité d'un pic correspondant à une masse de 4207 Da résultant de spectres de masse obtenus sur une lame de tissu déposée sur un support de référence et sur un support nanoporeux.
La figure 5B représente une image, réalisée avec un scanner, d'une lame de tissu déposée sur un support de référence et sur un support nanoporeux.
La figure 5C représente une distribution spatiale de l'intensité d'un pic correspondant à une masse de 7080 Da résultant de spectres de masse obtenus sur une lame de tissu déposée sur un support de référence et sur un support nanoporeux.
Les figures 6A et 6B sont des exemples de segmentation spatiale hiérarchique d'une image spectrale en considérant respectivement une lame de tissu déposée sur un support de référence et un tissu déposé sur un support nanoporeux.
La figure 7 montre une comparaison d'un spectre obtenu respectivement avec une lame de tissu déposée sur un support de référence et un tissu déposé sur un support nanoporeux.
La figure 8 représente un histogramme des coefficients de variation des pics détectés sur trois analyses consécutives de coupes prélevées d'un même échantillon.

### EXPOSE DE MODES DE REALISATION PARTICULIERS

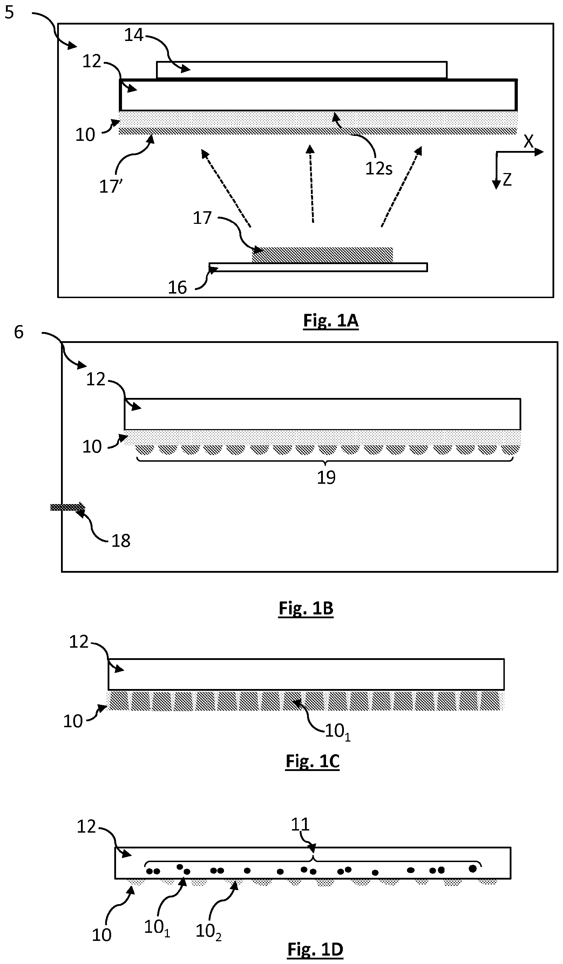

On a représenté, sur la figure 1A, une lame de tissu corporel 10, déposée sur la surface 12s d'un support nanoporeux 12, l'ensemble étant maintenu dans une enceinte 5. L'épaisseur de la lame de tissu est de préférence inférieure à 50 µm, et est notamment comprise entre 4 µm et 20 µm. Il s'agit d'une coupe histologique classique, obtenue à partir d'un tissu congelé ou d'un tissu fixé en paraffine.

Le support nanoporeux est constitué de pores, dont le diamètre, ou la plus grande dimension, parallèlement à la surface 12s du support, est compris entre 1 nm et 100 nm. La porosité est supérieure à 10%, et est de préférence comprise entre 30% et 80% selon une profondeur s'étendant, à partir de la surface 12s, entre 10 nm et 100 µm, voire davantage. La porosité désigne le volume occupé par les pores relativement au volume du support dans la profondeur considérée. Le support peut être un support de silicium poreux, tel que décrit dans la demande WO2013/098703, ou tout autre support présentant une surface fonctionnalisée et permettant la capture d'espèces biologique, par exemple par fonctionnalisation chimique.

La surface 12s peut être poreuse, fonctionnalisée et poreuse, ou fonctionnalisée et non poreuse. Par fonctionnalisée, on entend ayant subi un traitement de surface, de façon à présenter une affinité vis-à-vis d'une espèce biologique d'intérêt de façon à la capturer. Le terme espèce biologique désigne une espèce présente dans un tissu biologique, et libérée du tissu suite à une lyse de ce dernier. Il peut notamment s'agir d'une espèce endogène, par exemple une protéine ou un peptide, un exosome ou autre molécule naturellement présente dans le tissu. Il peut également s'agir d'une espèce biologique exogène, par exemple une molécule formant un agent thérapeutique, ou un marqueur, le marqueur pouvant être organique ou inorganique. D'une façon générale, les espèces biologiques intéressant l'invention ont une taille (diamètre ou plus grande dimension), ou sont inscrites dans un diamètre inférieur à quelques nanomètres, par exemple inférieur à 10 nm ou 20 nm.

Les procédés de fonctionnalisation de surface sont connus : il s'agit par exemple de déposer une couche fonctionnelle permettant une capture sélective de certaines espèces. La couche fonctionnelle peut être une couche chimique, comportant par exemple un matériau en C-18, propice à la capture de peptides et petites protéines, ou une couche anionique ou cationique. Il peut également s'agir d'une couche permettant une capture sélective par greffage antigène-anticorps.

La surface 12s s'étend selon un plan XY, les dimensions dans ce plan étant par exemple de 7,5x2.5cm ce qui correspond aux dimensions d'une lame de microscope de format standard.

Le support nanoporeux peut être mésoporeux, ce qui désigne usuellement des pores dont le diamètre est compris entre 2 nm et 50 nm. D'une façon générale, le terme nanoporeux désigne un matériau, de préférence de composition homogène, présentant des pores dont le diamètre moyen est inférieur à 1 µm (1 micromètre), et le plus souvent inférieur à 100 nm. Les matériaux mésoporeux présentent les dimensions correspondant le mieux aux applications visées, c'est-à-dire la capture de petites protéines ou de peptides.

Dans les exemples expérimentaux décrits ci-après, le support nanoporeux est obtenu à partir d'un substrat silicium 100 dopé au bore, de diamètre 200 mm, ayant fait l'objet d'une anodisation électrochimique dans une solution d'acide fluorhydrique (HF). L'électrolyte utilisé lors de l'anodisation comporte 3 volumes d'HF, 3 volumes d'isopropanol (IPA) et 4 volumes d'eau. La résistivité du silicium est comprise entre 10 et 20 mΩ.cm⁻¹. Le diamètre moyen des pores est de 15 nm, et ils s'étendent selon une profondeur de 1 µm. Il s'agit d'un support mésoporeux.

D'autres procédés de fabrication d'un substrat nanoporeux sont envisageables, par exemple un procédé de lithographie par faisceau d'électrons.

Le support nanoporeux 12 est disposé contre un refroidisseur 14, de façon à maintenir le support 12 et, par conduction thermique, le tissu 10, à une température inférieure à la température ambiante de l'enceinte 5. La température du support peut par exemple être de l'ordre de 0°C à 4°C. Le refroidisseur 14, ou générateur de froid, peut être un générateur thermoélectrique, par exemple un générateur à effet Peltier, un circuit fluidique thermorégulé ou un doigt froid relié à un cryostat. Le maintien du support 12 à basse température favorise des effets de condensation décrits ci-après.

L'enceinte 5 comporte également un radiateur 16, sur lequel est disposé un réactif de lyse 17 à l'état solide, par exemple sous la forme d'une poudre. La pression dans l'enceinte est ajustée de telle sorte que sous l'effet d'une augmentation de la température du radiateur 16, le réactif de lyse est sublimé. La sublimation du réactif de lyse 17 est matérialisée, sur la figure 1A, par des flèches en pointillés.

Une partie du réactif de lyse sublimé entre en contact avec le tissu 10, maintenu à basse température par le support 12. Il en résulte une condensation solide, ou cristallisation, du réactif de lyse 17 sur le tissu 10. La cristallisation forme des cristaux 17' fins et répartis de façon relativement homogène sur la surface du tissu. La taille de ces cristaux est également relativement uniforme, et peut être par exemple voisine de 100 nm. On forme ainsi un dépôt sec relativement homogène à la surface du tissu 10.

Le réactif de lyse 17 peut être de l'urée, ou comporter majoritairement de l'urée, le réactif étant déposé sur le radiateur 16 sous la forme d'une poudre. Lorsque la pression à l'intérieur de l'enceinte est de 2.10⁻³ mbar, et que le radiateur est porté à une température comprise entre 90°C et 120°C, l'urée est sublimée dans l'enceinte.

L'urée constitue le réactif de lyse préféré, les inventeurs ayant constaté des résultats optimaux avec ce réactif de lyse, mais d'autres réactifs de lyse ont envisageables, par exemple, des détergents, de type Triton (marque déposée) X 100 ((C₁₄H₂₂O(C₂H₄O)*ₙ*), Tween (marque déposée) 2° ou des actifs zwitterioniques (HEPES - acide 4-(2-hydroxyéthyl)-1-pipérazine éthane sulfonique) ou des enzymes.

Après dépôt du réactif de lyse sur le tissu 10, le support 12 est placé dans une chambre 6 dont l'atmosphère est riche, voire saturée en un solvant 18 en phase vapeur. Le choix du solvant dépend du réactif de lyse 17 préalablement utilisé. Lorsque le réactif de lyse 17 est de l'urée, le solvant 18 peut être une solution aqueuse de bicarbonate d'ammonium (50 mM), ou une solution aqueuse comportant du méthanol (par exemple 30% en volume de méthanol - 70% en volume d'eau). Il peut également s'agir d'une solution de méthanol avec une concentration de 50 mM de bicarbonate d'ammonium. La température ambiante de la chambre 6 est portée entre 30°C et 95°C, de façon à augmenter la quantité de solvant 18 présent en phase vapeur. Le solvant en phase vapeur se condense à la surface du tissu 10, en formant des gouttelettes 19 de réactif de lyse solubilisé sur cette dernière. Le diamètre de chaque gouttelette 19 est de préférence inférieur à 50 µm, et est de préférence inférieur à 20 µm. Il peut en particulier être compris entre 5 µm et 20 µm. Les gouttelettes sont de préférence espacées les unes des autres. Les cristaux du réactif de lyse sont solubilisés par les gouttelettes 19 condensées sur le tissu 10. Chaque gouttelette 19 constitue ainsi un microréservoir de réactif de lyse solubilisé. Afin de favoriser la condensation du solvant 18, le refroidissement du support 12 peut être maintenu.

La condensation du solvant est contrôlée de façon que les gouttelettes 19 de réactif de lyse solubilisé ne soient pas trop grandes, de façon à éviter que des gouttelettes 19 adjacentes coalescent. Les paramètres contrôlant la formation et la taille des gouttelettes 19 de solvant sont notamment la température dans la chambre, la température du tissu ainsi que la durée de l'exposition du tissu au solvant. Ces paramètres sont ajustés de façon à obtenir des gouttelettes 19 de taille préférentiellement inférieures à 50 µm, séparées les unes des autres, tout en étant suffisamment rapprochées les unes des autres. En effet, au niveau de chaque gouttelette élémentaire 19, le réactif de lyse 17 est solubilisé et imprègne le tissu 10, comme illustré en lien avec la figure 1C. Ainsi, au niveau de chaque gouttelette 19, le tissu subit une lyse localisée. Chaque goutte élémentaire 19 entraîne la formation d'un site de lyse 10₁, dans lequel le tissu est lysé. Dans chaque site de lyse 10₁, le matériel biologique présent dans le tissu est libéré, et peut atteindre la surface 12s du support nanoporeux 12. Le support 12 agit en tant que support de capture à l'égard de certaines espèces biologiques, dites espèces biologiques d'intérêt, ces dernières étant retenues au niveau des pores du support 12.

La répartition des gouttelettes 19 à la surface du tissu entraîne une répartition similaire des sites de lyse dans le tissu, chaque site de lyse étant séparé l'un de l'autre. Chaque site de lyse 10₁ s'étend entre chaque gouttelette 19 et support de capture 12. Deux sites de lyse adjacents sont séparés par du tissu 10₂ dit résiduel, comprenant du tissu non lysé ou du tissu lysé dans une moindre mesure qu'au niveau d'un site de lyse 10₁. Un point important de l'invention est d'obtenir une distribution bidimensionnelle des sites de lyse 10₁ selon un plan XY selon lequel s'étend la coupe de tissu, et présentant une résolution spatiale suffisante. De préférence, la répartition des sites de lyse est aussi régulière que possible. Une répartition régulière est obtenue lorsque les gouttelettes 19 formées à la surface du tissu sont régulièrement espacées et ont sensiblement la même taille. Par sensiblement la même taille, on entend la même taille, une tolérance de +/- 50% étant admise.

La distribution des sites de lyse 10₁ dans le tissu 10 permet d'obtenir une lyse dite spatialisée, suivant la répartition spatiale de chaque gouttelette 19 de solvant préalablement déposée à la surface du tissu. Cette spatialisation de la lyse entraîne une capture spatialisée d'espèces biologiques d'intérêt, au niveau de chaque site de lyse. En effet, sous l'effet de la lyse, des espèces biologiques 11 sont libérées par le tissu au niveau de chaque site de lyse 10₁. Elles sont canalisées par les parties résiduelles 10₂, et migrent vers le support de capture 12, comme illustré sur la figure 1D.

Le support 12 est ensuite rincé, de façon à éliminer les espèces biologiques non capturées. Après rinçage, le support 12 peut faire l'objet d'une analyse spatialement résolue, de façon à caractériser les espèces biologiques capturées. Comme précédemment indiqué, la taille de chaque site de lyse 10₁ dépend de la taille des gouttelettes 19 précédemment décrites. Les inventeurs ont estimé que la taille des gouttelettes peut être comprise entre 5 et 50 µm. Plus les gouttelettes 19 sont de grande taille, plus la lyse est efficace, ce qui favorise la sensibilité de la mesure, au détriment de la résolution spatiale. Lorsque les gouttelettes 19 sont de petite taille, par exemple comprise entre 5 et 15 µm, la résolution spatiale est optimale, mais la sensibilité de la mesure est réduite. Les inventeurs considèrent qu'il est préférable que la taille des gouttelettes 19 soit comprise entre 10 et 30 µm, de façon à privilégier la résolution spatiale.

Ainsi, un point clef du procédé est de permettre une lyse spatialisée, en regard d'un support de capture fonctionnalisé permettant une capture de certaines espèces biologiques, préalablement à une analyse spatialement résolue. Cela permet de remonter à une distribution spatiale des espèces biologiques 11 capturées dans le tissu 10. Si la lyse n'était pas spatialisée, un mélange pourrait se produire au cours du processus de lyse, et l'information de localisation, dans le tissu, des espèces capturées, serait perdue.

L'analyse spatialement résolue de l'échantillon peut notamment être une spectrométrie de masse avec désorption laser, par exemple de type MALDI ou SELDI (Surface Enhanced Laser Desorption lonization).

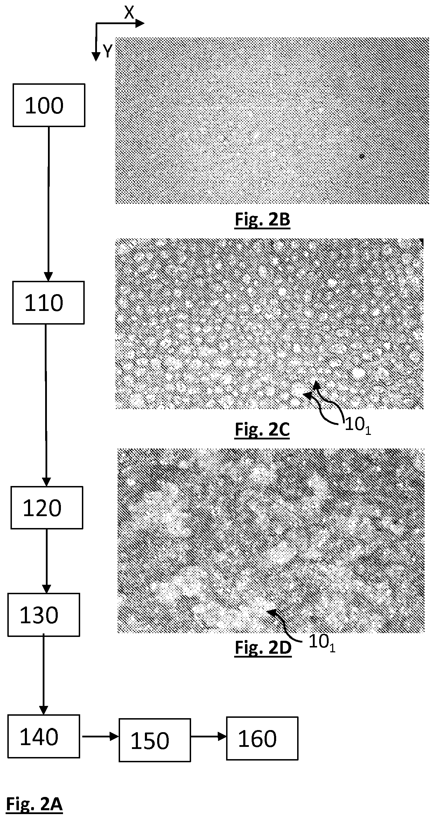

La figure 2A représente les principales étapes du procédé, décrites en lien avec les figures 2B, 2C et 2D, ces dernières étant des images d'une lame de tissu observée au microscope (grandissement optique x10).

Etape 100 : disposition du tissu face au réactif de lyse. La figure 2B représente une image d'une coupe de tissu testée. Il s'agit d'une coupe, d'épaisseur 10 µm, obtenue à partir d'un prélèvement d'un cerveau de rat cryogénisé à -20°C. Après découpe, la coupe est séchée pendant 30 minutes, lavée par de l'éthanol (entre 70% et 100% en volume) durant 30 secondes, puis trempée dans un fixateur (solution de Carnoy, connue de l'homme du métier), pendant 2 minutes, puis à nouveau rincée à l'éthanol.

Etape 110 : application d'un réactif de lyse 17 par sublimation du réactif de lyse et condensation sur le tissu.

Etape 120 : solubilisation du réactif de lyse : il s'agit de mettre en contact le réactif de lyse 17, réparti à la surface du tissu 10, avec un solvant 18, par exemple en phase vapeur. Cette étape peut également être désignée par le terme "incubation".

Etape 130 : lyse spatialisée du tissu 10, entraînant une capture spatialisée d'espèces biologiques 11 par le support de capture 12.

Etape 140 : rinçage de la surface 12s du support 12, par trois bains d'eau distillée d'une minute chacun, de façon à éliminer les parties résiduelles de tissu non lysé. Les inventeurs ont constaté que l'urée s'éliminait très bien par rinçage simple contrairement à d'autres agents de lyse, ces derniers laissant des traces pouvant induire une contamination de l'analyse moléculaire par spectrométrie de masse.

Le procédé peut alors comporter une étape 150 de traitement en vue d'une analyse par spectrométrie de masse par désorption laser. Il s'agit de déposer une matrice organique, par exemple par pulvérisation. Le procédé comporte également une étape 160 d'analyse par la spectrométrie de masse, de façon à obtenir une distribution spatiale de spectres de masse, ainsi obtenir une information spectrale représentative de chaque partie élémentaire lysée 10₁.

La figure 2C est une image d'une coupe de tissu, similaire à celle représentée sur la figure 2B, après mise en œuvre des étapes 100 à 130, préalablement décrites. On observe des sites de lyse 10₁ répartis selon un maillage bidimensionnel à la surface du tissu. Les sites de lyse correspondent aux parties les plus claires de l'image. Chaque site de lyse 10₁ est délimité par une partie résiduelle 10₂, correspondant aux contours sombres.

Selon une variante, lors de l'étape 110, le dépôt du réactif de lyse 17 sur le tissu n'est pas réalisé par sublimation, mais par pulvérisation. Cela entraîne la formation de gouttelettes 17' sur le tissu 10. La taille des gouttelettes pulvérisées peut être comprise entre 10 µm et 20 µm. Un exemple de réactif de lyse est une solution aqueuse d'urée, de molarité comprise entre 4M et 8M. Selon cette variante, au cours de l'étape 120, les gouttelettes de réactifs de lyse s'imprègnent de solvant, formant des gouttelettes 19 de réactif de lyse solubilisé.

Selon une autre variante, lors de l'étape 120, le solvant 18 est appliqué sur le tissu en étant pulvérisé, de façon à former des gouttelettes 19 de diamètre préférentiellement inférieur à 50 µm à la surface du tissu.

La figure 2D représente une image d'un tissu, similaire à celui représenté sur la figure 2B, après pulvérisation du réactif de lyse, puis pulvérisation du solvant en phase vapeur. Les parties claires correspondent à des sites de lyse 10₁. Cette image a été obtenue avec le même grossissement que la figure 2C. Les sites de lyse sont plus grands et moins nettement délimités que sur l'image 2C.

Les inventeurs ont constaté que le dépôt du réactif de lyse par pulvérisation permet d'obtenir une meilleure efficacité de lyse, conduisant à une augmentation de la sensibilité, tandis qu'un dépôt de réactif de lyse par sublimation permet d'obtenir une meilleure résolution spatiale. Cela est dû au fait que la sublimation permet la formation de gouttelettes 19 plus petites que celles obtenues par pulvérisation.

### Essais expérimentaux.

Des essais expérimentaux ont été réalisés sur des lames de cerveau de rat d'épaisseur 10 µm, telles que précédemment décrites. Le cerveau prélevé comportait deux zones tumorales. Les essais ont été réalisés en considérant d'une part un support nanoporeux (désigné par l'acronyme MPSi) et d'autre part un support d'ITO (oxyde d'indium-étain), formant un support de référence. Les tissus déposés sur chaque lame sont comparables, et sont issus du même prélèvement. Les tissus ont fait l'objet d'une analyse par spectrométrie de masse MALDI-TOF (Time of Flight) en mode linéaire avec une tension d'accélération de +20 kV à l'aide d'un dispositif Ultraflex III MALDI TOF/TOF (Bruker Daltonics) équipé d'un laser opérant selon une fréquence impulsionnelle de 1000 Hz. Les spectres ont été acquis selon une plage de ratio masse sur charge comprise entre 1000 et 20000 Da (Dalton). La résolution latérale est de 75 µm, avec 300 impacts laser par point. Préalablement à l'acquisition des spectres, les échantillons ont été recouverts d'une matrice organique, appliquée par pulvérisation. La matrice utilisée est un mélange de méthanol/eau/acide trifluoroacétique, avec des fractions volumiques respectives de 50/49.8/0.2. Les spectres discutés ci-après sont normalisés par le courant ionique total, c'est-à-dire l'intégrale des canaux du spectre.

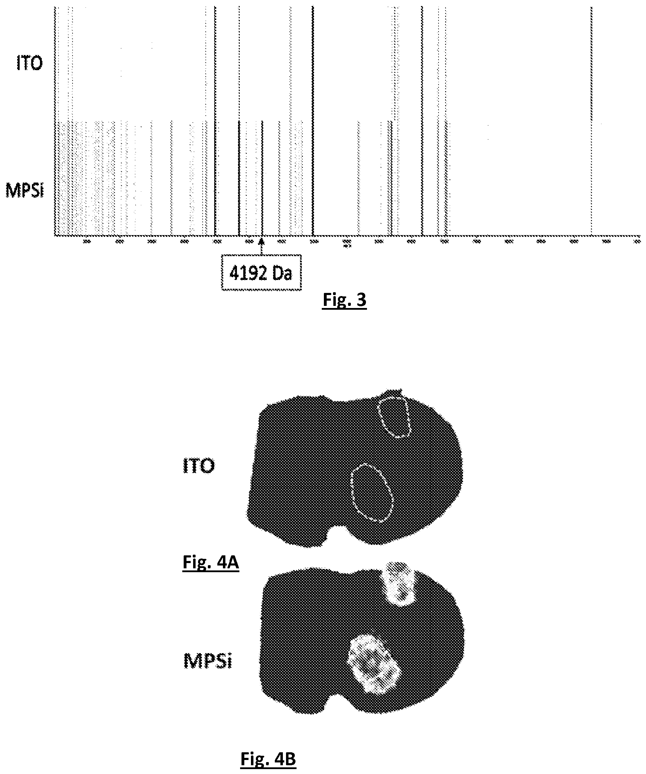

La figure 3 représente un spectre, une bande spectrale comprise entre 1000 et 20000 Da, acquis avec un support de référence (ITO), préparé selon un procédé standard, ainsi qu'en mettant en œuvre l'invention, sur un support de capture en silicium nanoporeux (MPSi). On observe que le spectre acquis à l'aide du support nanoporeux comporte davantage de pics : l'information spectrale est donc plus riche, et susceptible de faire apparaître des traceurs non décelables avec le support classique d'ITO.

Les figures 4A et 4B représentent, pour chaque point du tissu (chaque point représentant un pixel de 75 µm de 75 µm), l'intensité du pic de 4192 Da. La figure 4A correspond au support ITO, tandis que la figure 4B correspond au support nanoporeux. La figure 4A comporte un signal assimilable à du bruit, tandis que la figure 4B permet de localiser la présence de ce pic au niveau des zones tumorales délimitées par des pointillés. Un tel pic peut donc correspondre à une signature spectrale d'une tumeur, clairement observable à l'aide de l'invention.

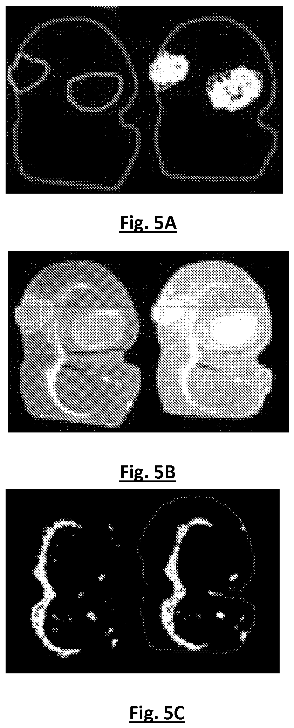

La figure 5A représente une image spectrale réalisée à 4207 Da, avec le support ITO à droite et le support nanoporeux à gauche. On observe à nouveau que l'invention permet d'obtenir davantage d'information spécifique à la tumeur.

La figure 5B représente des images optiques des tissus, réalisées à l'aide d'un scanner, dont les images spectrales ont été représentées sur les figures 4A, 4B et 5A. La partie gauche de l'image représente le tissu déposé sur un support ITO. La partie droite de l'image représente le tissu déposé sur le support de silicium nanoporeux. On observe bien l'anatomie du crâne, et la présence des tumeurs.

La figure 5C représente des images spectrales réalisées à 7080 Da, avec le support ITO (partie gauche) et le support nanoporeux (partie droite). Le pic à 7080 Da permet d'observer le corps calleux sur chaque image. La figure 5C permet de vérifier la cohérence des résultats obtenus avec les deux supports sur une bande spectrale de référence connue.

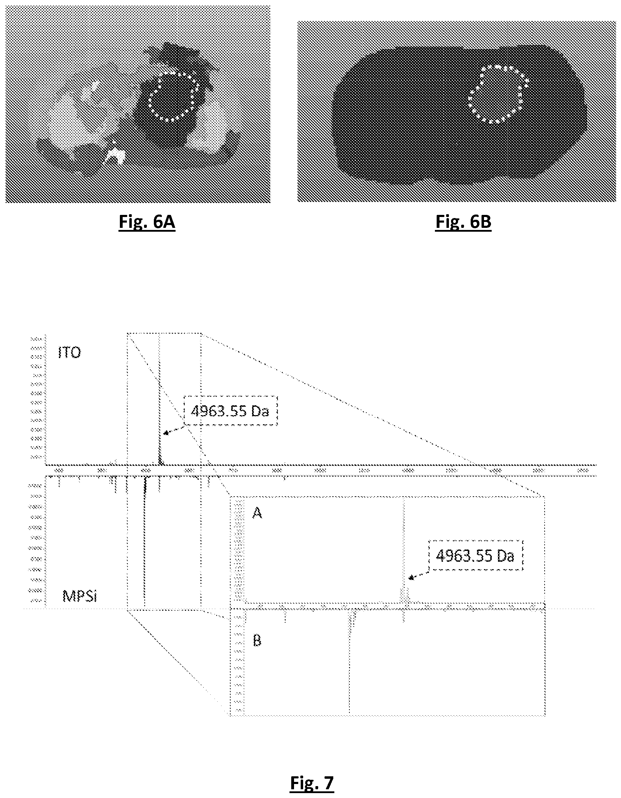

Les figures 6A et 6B montrent un exemple de classification spectrale hiérarchique réalisée respectivement avec le support d'ITO et le support nanoporeux. Cette classification permet de regrouper les différentes zones spatiales au niveau desquelles des spectres similaires ont été acquis. Ainsi, le tissu examiné subit une partition en différentes zones spatiales, en fonction d'un critère de similitude de spectre. La classification peut être réalisée de façon itérative, de façon à obtenir une classification représentative des caractéristiques anatomiques du tissu examiné.

Sur la figure 6A, plusieurs classes de spectres ont été définies. Les contours de la tumeur apparaissent de façon approximative. Cette classification a nécessité cinq itérations successives. Sur la figure 6B, l'algorithme de classification a déterminé deux classes, l'une correspondant à la partie saine, l'autre correspondant à la partie tumorale. Cette classification a été obtenue en une seule itération. L'invention permet d'obtenir une meilleure discrimination entre un spectre acquis sur une zone tumorale et un spectre acquis sur une zone saine.

La figure 7 représente le spectre moyen représentant la moyenne de tous les spectres acquis sur l'ensemble de la coupe histologique de cerveau respectivement avec un support ITO et un support nanoporeux (MPSi), les deux supports ayant subi le même protocole de traitement. On observe que le spectre moyen obtenu avec le support nanoporeux comprend un pic à 4963.55 Da, correspondant au peptide Thymosine Beta 4, fortement diminué par rapport au spectre moyen obtenu avec le support nanoporeux 12. Il s'agit d'un peptide abondant, dont la présence peut masquer la détection de petites protéines ou d'autres peptides. Son atténuation sur le spectre obtenu avec le support nanoporeux est donc avantageuse. Les parties A et B de la figure correspondent à des zooms des spectres respectivement acquis à l'aide du support ITO et du support nanoporeux, le zoom correspondant à une région d'intérêt spectrale autour de 4963.55 Da.

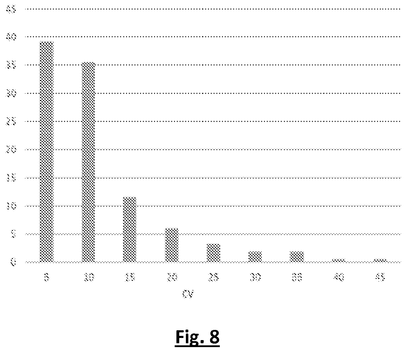

La reproductibilité du procédé a été testée. Trois coupes correspondant au même prélèvement ont été déposées sur un support de silicium nanoporeux similaire, et ont fait l'objet d'une préparation et d'une analyse spectrale telles que préalablement décrites. Un spectre moyen a été mesuré sur chaque coupe, les trois spectres moyens obtenus comportant 350 pics en commun. Le coefficient de variation d'intensité de chaque pic a été calculé sur les trois spectres moyens. La figure 8 représente un histogramme des coefficients de variation CV L'axe des abscisses représente la valeur d'un coefficient de variation, et l'axe des ordonnées représente le nombre de pics. On observe que 90% des pics détectés sur les trois coupes ont un coefficient de variation inférieur à 15%. Ces résultats attestent d'une bonne reproductibilité du procédé. L'invention pourra être mise en œuvre comme aide au diagnostic ou comme outil de recherche, de façon à déterminer des traceurs spécifiques de pathologies particulières.

## Revendications

1. Procédé de capture d'espèces biologiques à partir d'un tissu biologique, comportant :
a) l'application d'un tissu biologique (10) sur un support de capture (12), le support de capture étant apte à capturer sélectivement au moins une espèce biologique (11), dite espèce biologique d'intérêt ;
le procédé étant **caractérisé en ce qu'**il comporte également les étapes suivantes :
b) dépôt d'un réactif de lyse (17) sur le tissu biologique ;
c) mise en contact du réactif de lyse, ainsi déposé, avec un solvant (18), de façon à former des gouttelettes (19), espacées les unes des autres, à la surface du tissu, les gouttelettes comportant le réactif de lyse (17) solubilisé par le solvant (18);
d) formation de sites de lyse (10₁), chaque site de lyse correspondant à une portion du tissu (10) s'étendant entre une gouttelette (19) et le support de capture (12) et, dans chaque site de lyse, lyse du tissu biologique (10) par le réactif de lyse solubilisé par le solvant, la lyse entraînant une libération puis une capture d'espèces biologiques d'intérêt (11) du tissu par le support de capture ;
e) rinçage du support de capture (12), pour éliminer les espèces biologiques non capturées.

2. Procédé selon la revendication 1, dans lequel le procédé comporte une étape f) d'analyse spatialement résolue du support de capture (12), de manière à obtenir une information relative à la répartition spatiale des espèces biologiques d'intérêt capturées lors de l'étape d).

3. Procédé selon la revendication 2, dans lequel lors de l'étape f) l'analyse mise en œuvre est une spectrométrie de masse spatialement résolue.

4. Procédé selon l'une quelconque des revendications précédentes, dans lequel lors de l'étape b), le réactif de lyse (17) est pulvérisé sur le tissu biologique (10).

5. Procédé selon l'une quelconque des revendications 1 à 3, dans lequel lors de l'étape b), le réactif de lyse (17) se condense sur le tissu biologique (10), de façon à former des dépôts solides sur ce dernier.

6. Procédé selon la revendication 5, comportant une sublimation du réactif de lyse (17), de telle sorte que le réactif de lyse (17), à l'état gazeux, se condense sur le tissu biologique (10).

7. Procédé selon l'une quelconque des revendications précédentes, dans lequel lors de l'étape c), les gouttelettes (19) sont formées par condensation du solvant (18) sur le tissu.

8. Procédé selon l'une quelconque des revendications 1 à 6, dans lequel lors de l'étape c), les gouttelettes (19) sont formées par pulvérisation du solvant (18) sur le tissu.

9. Procédé selon l'une quelconque des revendications précédentes, dans lequel le support de capture (12) est un support nanoporeux, comportant des pores dont le diamètre ou la plus grande taille est inférieur à 1 µm.

10. Procédé selon l'une quelconque des revendications précédentes, dans lequel le support de capture (12) est en silicium.

11. Procédé selon l'une quelconque des revendications précédentes, dans lequel l'épaisseur du tissu biologique (10) est inférieure à 50 µm ou inférieure à 20 µm.

## Patentansprüche

1. Verfahren zum Einfangen biologischer Spezies aus einem biologischen Gewebe, umfassend:
a) das Aufbringen eines biologischen Gewebes (10) auf einen Einfangträger (12), wobei der Einfangträger dafür ausgelegt ist, selektiv mindestens eine so genannte interessierende biologische Spezies (11) einzufangen,
wobei das Verfahren **dadurch gekennzeichnet ist, dass** es außerdem die folgenden Schritte umfasst:
b) das Aufbringen eines Lysereagenzes (17) auf das biologische Gewebe,
c) das In-Kontakt-Bringen des so aufgebrachten Lysereagenzes mit einem Lösungsmittel (18), so dass sich voneinander beabstandete Tröpfchen (19) auf der Oberfläche des biologischen Gewebes bilden, wobei die Tröpfchen das durch das Lösungsmittel (18) solubilisierte Lysereagenz (17) umfassen,
d) das Bilden von Lysestellen (10₁), wobei jede Lysestelle einem Abschnitt des Gewebes (10) entspricht, der sich zwischen einem Tröpfchen (19) und dem Einfangträger (12) erstreckt, und an jeder Lysestelle die Lyse des biologischen Gewebes (10) durch das Lysereagenz, das durch das Lösungsmittel solubilisiert ist, wobei die Lyse eine Freisetzung und anschließendes Einfangen der interessierenden biologischen Spezies (11) aus dem Gewebe durch den Einfangträger bewirkt,
e) das Spülen des Einfangträgers (12), um die nicht eingefangenen biologischen Spezies zu beseitigen.

2. Verfahren nach Anspruch 1, wobei das Verfahren einen Schritt f) der räumlich aufgelösten Analyse des Einfangträgers (12) umfasst, so dass Informationen hinsichtlich der räumlichen Verteilung der im Schritt d) eingefangenen interessierenden biologischen Spezies erhalten werden.

3. Verfahren nach Anspruch 2, wobei es sich im Schritt f) bei der durchgeführten Analyse um eine räumlich aufgelöste Massenspektrometrie handelt.

4. Verfahren nach einem der vorhergehenden Ansprüche, wobei im Schritt b) das Lysereagenz (17) auf das biologische Gewebe (10) gesprüht wird.

5. Verfahren nach einem der Ansprüche 1 bis 3, wobei im Schritt b) das Lysereagenz (17) auf dem biologischen Gewebe (10) kondensiert, so dass es feste Ablagerungen auf diesem bildet.

6. Verfahren nach Anspruch 5, umfassend eine Sublimation des Lysereagenzes (17), so dass das Lysereagenz (17) im gasförmigen Zustand auf dem biologischen Gewebe (10) kondensiert.

7. Verfahren nach einem der vorhergehenden Ansprüche, wobei im Schritt c) die Tröpfchen (19) durch Kondensation des Lösungsmittels (18) auf dem Gewebe gebildet werden.

8. Verfahren nach einem der Ansprüche 1 bis 6, wobei im Schritt c) die Tröpfchen (19) durch Sprühen des Lösungsmittels (18) auf das Gewebe gebildet werden.

9. Verfahren nach einem der vorhergehenden Ansprüche, wobei es sich bei dem Einfangträger (12) um einen nanoporösen Träger handelt, der Poren umfasst, deren Durchmesser oder größte Abmessung kleiner als 1 µm ist.

10. Verfahren nach einem der vorhergehenden Ansprüche, wobei der Einfangträger (12) aus Silizium besteht.

11. Verfahren nach einem der vorhergehenden Ansprüche, wobei die Dicke des biologischen Gewebes (10) kleiner als 50 µm oder kleiner als 20 µm ist.

## Claims

1. A method for capturing biological species from a biological tissue, comprising :
a) application of a biological tissue (10) onto a capture support (12), the capture support being capable of selectively capturing at least one biological species (11), referred to as the biological species of interest;
the method being **characterized in that** it comprises the following steps
b) deposition of a lysis reagent (17) onto the biological tissue;
c) bringing of the lysis reagent thus deposited into contact with a solvent (18), so as to form droplets (19), spaced out from one another, at the surface of the tissue, the droplets comprising the lysis reagent (17) solubilized in the solvent (18);
d) formation of lysis sites (10₁), each lysis site corresponding to a portion of the tissue (10) extending between a droplet (19) and the capture support (12) and, in each lysis site, lysis of the biological tissue (10) by the lysis reagent solubilized by the solvent, the lysis bringing about a release and then a capture of biological species of interest (11) of the tissue by the capture support;
e) rinsing of the capture support (12), in order to remove the uncaptured biological species.

2. The method as claimed in claim 1, wherein the method comprises a step f) of spatially resolved analysis of the capture support (12), so as to obtain information relating to the spatial distribution of the biological species of interest captured during step d).

3. The method as claimed in claim 2, wherein, during step f), the analysis carried out is spatially resolved mass spectrometry.

4. The method as claimed in any one of the preceding claims, wherein, during step b), the lysis reagent (17) is sprayed onto the biological tissue (10).

5. The method as claimed in any one of claims 1 to 3, wherein, during step b), the lysis reagent (17) condenses on the biological tissue (10), so as to form solid deposits on said tissue.

6. The method as claimed in claim 5, comprising a sublimation of the lysis reagent (17) such that the lysis reagent (17), in the gaseous state, condenses on the biological tissue (10).

7. The method as claimed in any one of the preceding claims, wherein, during step c), the droplets (19) are formed by condensation of the solvent (18) on the tissue.

8. The method as claimed in any one of claims 1 to 6, wherein, during step c), the droplets (19) are formed by spraying the solvent (18) onto the tissue.

9. The method as claimed in any one of the preceding claims, wherein the capture support (12) is a nanoporous support, comprising pores of which the diameter or the largest size is less than 1 µm.

10. The method as claimed in any one of the preceding claims, wherein the capture support (12) is made of silicon.

11. The method as claimed in any one of the preceding claims, wherein the thickness of the biological tissue (10) is less than 50 µm or less than 20 µm.
